# EUROPÄISCHE PATENTSCHRIFT

(11) **EP 3 142 641 B1**
(45) Veröffentlichungstag und Bekanntmachung des Hinweises auf die Patenterteilung: **25.11.2020**
(21) Anmeldenummer: 15734557.0
(22) Anmeldetag: 12.05.2015
(51) Int. Cl.: A61K 31/164, A61K 9/00, A61K 47/14, A61K 47/44, A61K 47/32, A61K 31/4164, A61P 5/14, A61P 5/16, A61K 31/355

(54) **FORMULIERUNGEN ZUR BEHANDLUNG VON HYPERTHYREOSE**
FORMULATIONS FOR TREATMENT OF HYPERTHYROIDISM
FORMULATIONS POUR TRAITER L'HYPERTHYROÏDIE

(30) Priorität: 13.05.2014 DE 102014106749
(43) Veröffentlichungstag der Anmeldung: 22.03.2017
(73) Patentinhaber: DendroPharm GmbH, 12107 Berlin (DE)
(72) Erfinder: MORÉ, Sam, 01109 Dresden (DE)
(74) Vertreter: Moré, Solveig Helga
(86) Internationale Anmeldenummer: PCT/DE2015/100192
(87) Internationale Veröffentlichungsnummer: WO 2015/172769

(56) Entgegenhaltungen:
- CN-A- 1 692 940
- BUIJTELS J J C W M ET AL: "Transdermal carbimazole for the treatment of feline hyperthyroidism", TIJDSCHR. DIERGENEESKD,, Bd. 131, 1. Januar 2006 (2006-01-01), Seiten 478-482, XP008108982, in der Anmeldung erwähnt

## Beschreibung

Die vorliegende Erfindung betrifft Zusammensetzungen, gemäß den Ansprüchen, Z insbesondere zur Behandlung der Hyperthyreose, vor allem bei Katzen. Diese Zusammensetzungen sind zur transdermalen Verabreichung eines Wirkstoffs formuliert und können z.B. in der Ohrmuschel der Katze aufgetragen werden. Sie umfassend einen Wirkstoff wie Thiamazol (engl. Methimazole oder 2-Mercapto-1-methylimidazol) oder Carbimazol, mindestens ein Wachs, mindestens ein fettes Öl, und einen Emulgator, gemäß den Ansprüchen. Als

Emulgator wird ein Nanocarrier verwendet. Dieser ist ein unimolekularer Nanocarrier mit dendritischer Struktur, wobei der Nanocarrier aus einem dendritischen Kern und aus mindestens zwei Schalen besteht, wobei eine innere Schale mit einem ersten Linker mit dem dendritischen Kern verbunden ist und eine äußere Schale mit einem zweiten Linker mit der inneren Schale verbunden ist, gemäß den Ansprüchen.

In einer Ausführungsform wird die Zusammensetzung als Stift zur Verfügung gestellt, in einer weiteren als halbfeste Formulierung in einem geeigneten Applikator, der eine oder auch mehrere Dosiseinheiten enthalten kann.

Die Hyperthyreose ist eine krankhafte Überfunktion der Schilddrüse, die sich in einer übermäßigen Produktion von Schilddrüsenhormonen äußert, so dass im Organismus ein entsprechendes Überangebot entsteht. Infolgedessen kann es zu einer Vielzahl von Krankheitserscheinungen wie starker Schweißproduktion, beschleunigtem Herzschlag, Gewichtsverlust, Nervosität und Zittern kommen. Die häufigsten Ursachen der Hyperthyreose sind bei Menschen der Morbus Basedow und die Schilddrüsenautonomie, die beide mit einer erhöhten Sekretionsleistung der Schilddrüse einhergehen, und eine erhöhte Zufuhr von Schilddrüsenhormonen in Form von Medikamenten (Thyreotoxicosis factitia). Im Extremfall einer krisenhaften lebensbedrohlichen Überfunktion spricht man von einer "thyreotoxischen Krise" (Synonym *Thyreotoxikose,* "Schilddrüsenhormonvergiftung") (wikipedia).

Hyperthyreose kommt nicht nur bei Menschen vor, sondern auch bei Tieren. Es ist die häufigste endokrine Störung der Katze. Behandlungsoptionen sind Radioiodtherapie, Thyroidektomie oder medikamentöse Behandlung. Bei der medikamentösen Therapie werden den Betroffenen Thyreostatika, die die Bildung der Schilddrüsenhormone hemmen, solange und so hoch dosiert verschrieben, bis eine Normalisierung der Laborwerte (Euthyreose) erreicht wird. Häufig werden schwefelhaltige Thyreostatika (Propylthiouracil, Carbimazol, Thiamazol und andere) eingesetzt, die jedoch eine etwa einwöchige Wirklatenz aufweisen; ist ein schneller Wirkeintritt nötig, so kann Natriumperchlorat, das die Aufnahme von Jod in die Schilddrüse hemmt, eingesetzt werden. Thyreostatika sind bei einer Hyperthyreose, die durch eine Schilddrüsenentzündung (Thyreoiditis) ausgelöst wird, unwirksam, da sie die Freisetzung der in der Schilddrüse gespeicherten Hormone ("präformierte Hormone") durch den Entzündungsprozess nicht beeinflussen können, wirken aber gut bei anderen Ursachen der Hyperthyreose. Propranolol (ein nicht-kardioselektiver β-Rezeptorenblocker) kann bei Tachykardie ergänzend eingesetzt werden, mildert aber auch die nicht-kardialen Symptome der Hyperthyreose und hemmt die Umwandlung (Konversion) von T₄ zu T₃.

Radiotherapie ist teuer und irreversibel. Die oft ebenfalls vorhandene Nierenproblematik bei alten Katzen wird durch den Ausfall der Schilddrüse verschlimmert, daher ist eine gut eingestellte medikamentöse Behandlung, die eine variable Dosierung des Thiamazols/Carbimazols erlaubt, vorteilhafter, da so der Blutdruck leicht erhöht bleiben kann: die Niere wird besser durchspült. Die medikamentöse Behandlung ist in vielen Ländern die Behandlung der Wahl. Carbimazol, ein Thioharnstoff-Derivat, ist ein Prodrug und wird unmittelbar nach der Resorption in seine Wirkform Thiamazol umgewandelt.

Thiamazol und Carbimazol werden üblicherweise auch vor einer Bestrahlungstherapie oder seltener Thyroidektomie (v.a. bei Menschen) eingesetzt. Beide Wirkstoffe können in Katzen mit Niereninsuffizienz zur Langzeittherapie oder als Test eingesetzt werden, um herauszufinden, ob Serum-T₄ sicher durch eine Bestrahlung oder durch eine Thyroidektomie gesenkt werden kann, ohne zu einer schwer behandelbaren Niereninsuffizienz mit Nierenversagen zu führen. Thiamazol hemmt Thyroidperoxidase, ein Enzym, welches bei der Oxidation von lodid zu elementarem Iod, den Einbau von Iod in Thyreoglobulin und Einbau in Tyrosylreste sowie deren Kombination zu T₃ und T₄ eine Rolle spielt.

Typischerweise wird Thiamazol oral verabreicht, wobei bei Menschen eine einmal tägliche Gabe meist ausreicht. Bei Katzen wurde gezeigt, dass eine Verabreichung zweimal am Tag effektiver ist als einmal am Tag (Trepanier, 2007, Vet Clin Small Anim 37:775-788). Eine Behandlung mit Carbimazol muss ebenfalls täglich durchgeführt werden. Bei ca. 20% der behandelten Katzen führt die orale Verabreichung zu Nebenwirkungen, insbesondere Erbrechen, Lethargie, Juckreiz, Lebererkrankungen, Blutbildveränderungen), die nach Absetzen des Medikaments aber zumeist wieder verschwinden. Zu Erbrechen, Anorexie und Lethargie kommt es bei ca. 10% - 18% der oralen Verabreichung von Thiamazol. Transdermale Verabreichung von Thiamazol ist mit deutlich weniger gastrointestinalen Nebenwirkungen assoziiert als orale Verabreichung (Sartor et al., 2004, J Vet Intern Med 18:651-655). Das Medikament wird üblicherweise vom Besitzer in der inneren Ohrmuschel der Katze aufgetragen, wobei ein Kontakt mit der Haut des Halters vermieden werden sollte, so dass dieser i.A. Handschuhe oder Fingerlinge trägt. Bisher gibt es in der EU kein zugelassenes Carbimazol oder Thiamazol in einer transdermalen Formulierung.

Bisher wird Thiamazol transdermal vor allem in Pluronic Lecithin Organogel (PLO) verabreicht. PLO wirkt als Permeationsverbesserer durch das Stratum corneum. Obwohl gezeigt wurde, dass die Aufnahme von Thiamazol nach einer einzelnen Dosis in PLO transdermal schlecht war. (Hoffmann et al., 2002, J. vet. Pharmacol. Therap. 25:189-193), wirkt eine chronische transdermale Verabreichung und ist effektiv zur Verringerung der Serum-T₄-Konzentration (Lecuyer et al., 2006, Can Vet J 47:131-135, Hoffmann et al., 2003, Journal of Feline Medicine and Surgery 5:77-82).. Zu gastrointensinalen Nebenwirkungen kam es in einer randomisierten Studie nur bei 4% statt 24% (orale Verabreichung) der Katzen (Sartor et al., 2004, J Vet Intern Med 18:651-655). Nachteile der Verabreichung von Thiamazol in PLO sind schlechte Aufnahme, Rötungen am Auftragungsort, erhöhte Formulierungskosten und ungeklärte Stabilität der Zusammensetzung. (Sartor et al., 2004, J Vet Intern Med 18:651-655, Trepanier, 2007, Vet Clin Small Anim 37:775-788).

Im Withdrawal assessment report for Nexcyon Pharmaceuticals Ltd and Enthryv (EMEA/V/C/002808/0000) wird eine Formulierung von Thiamazol beschrieben. Verwendet wurde eine ethanolische Lösung von Thiamazol, die octisalate and padimate-O als Penetrationsverstärker bzw. Penetrationsmodulator enthielten. Die Dosierungsoptionen waren 0.25 ml (18.75 mg), 0.5 ml (37.5 mg) or 0.75 ml (56.25 mg Methimazole), die alle 2 bis 3 Tage transdermal appliziert werden sollten. Die aufgetragenen Mengen waren jeweils 0.25 ml, 0.5 ml and 0.75 ml. Die Effizienz der Behandlung wurde jedoch als nicht gleichwertig zur oralen Therapie beurteilt. Weiterhin war die Anwendersicherheit nicht gegeben, da die Thiamazolrückstände im Fell der Katze zu hoch waren.

Buijtels et al, 2006 (Tijdschrift voor Diergeneeskunde 131(13):478-482) beschreiben eine Formulierung zur transdermalen Verabreichung von Carbimazol in Augensalbe mit Lecithin. Die Verwendung von Lecithin ist jedoch nicht wünschenswert, da dieses die Formulierung klebrig und schwer aufzutragen macht und ferner keine guten Absorbtionscharakteristika aufweist.

US 2010/0137389 A1 beschreibt eine antihyperthyroidale Zusammensetzung zur transdermalen Verabreichung, welche mindestens ein aprotisches polares Lösungsmittel, mindestens ein Polyol, insbesondere PEG4000, und mindestens eine Fettsäure umfasst. Nachteile der in der Anmeldung beschriebenen Formulierungsbeispiele sind z.B. der hohe Gehalt an oxidationsempfindlicher Ölsäure, und der hohe Gehalt an Propylenglycol, der in den beschriebenen Mengen (> 35%) zu Hautreizungen führen kann. Weiterhin wird NMP als Penetrationsverstärker vorgeschlagen, hier besteht ebenfalls das Risiko von Hautreizungen.

Buijtels et al, 2006 und US 2010/0137389 A1 lehren, dass es oft schwer ist, für einen Wirkstoff eine Formulierung mit einem Penetrationsverbesserer, Penetrationsmodulator oder Schlepper zu finden, welcher passende Eigenschaften aufweist, da dies für jeden Wirkstoff und das gewünschte Drug-Release-Profil individuell getestet werden muss. Die Eigenschaften als Penetrationsverbesserer sind damit für jeden Wirkstoff bzw. jedes Drug-Release-Profil spezifisch oder zumindest nur für eng verwandte Wirkstoffe mit vergleichbaren physiko-chemischen Eigenschaften vorhersagbar.

Dem gegenüber stellten sich die Erfinder die Aufgabe, eine neue Zusammensetzung zur transdermalen Verabreichung von Thiamazol und/oder Carbimazol zur Verfügung zu stellen, welche bevorzugt zumindest einige dieser Nachteile überwindet. Weiterhin ist es aus Anwendersicht vorteilhaft, eine so gering wir Mögliche Menge sowohl von Wirkstoff als auch der den Wirkstoff enthaltenen Salbenformulierung zu applizieren. Dies ist darin begründet, dass bei der Wirkstoff teratogen ist und daher keine Aufnahme durch den Katzenhalters stattfinden darf. Das damit verbundene Risiko wird durch die kleinen Mengen d.h. 25-100 µl als typische Applizierungsmenge für Katzen mit durchschnittlich abgesenkten TT4 Wert gelöst, Diese sind um bis zu 75 % niedriger als die in der Literatur beschriebenen Werte für bisherige dermale Formulierungen. Diese Aufgabe wird durch die vorliegende Erfindung, insbesondere den Gegenstand der Ansprüche, gelöst.

Gegenstand der Erfindung ist eine Zusammensetzung, umfassend
a) einen Wirkstoff ausgewählt aus der Gruppe bestehend aus Thiamazol und/oder Carbimazol;
b) mindestens ein Bienenwachs, und
c) mindestens ein fettes Öl, ausgewählt aus der Gruppe umfassend Erdnussöl,
Mandelöl, Sonnenblumenöl, Leinöl, Olivenöl oder Nachtkerzenöl, sowie d) einen amphiphilen unimolekularen Nanocarrier gemäß den Ansprüchen.

Bevorzugt ist der Wirkstoff Thiamazol. Auch Carbimazol kann verwendet werden, welches als Prodrug von Thiamazol wirkt.

Wachse sind Stoffe, die durch ihre mechanisch-physikalischen Eigenschaften definiert werden. Ihre chemische Zusammensetzung und Herkunft sind hingegen sehr unterschiedlich. Ein Stoff wird als Wachs bezeichnet, wenn er bei 20 °C knetbar, fest bis brüchig hart ist, eine amorphe bis feinkristalline Struktur aufweist, farblich durchscheinend bis opak, aber nicht glasartig ist, über 40 °C ohne Zersetzung schmilzt, wenig oberhalb des Schmelzpunktes leicht flüssig (wenig viskos) ist, eine stark temperaturabhängige Konsistenz und Löslichkeit aufweist sowie unter leichtem Druck polierbar ist. Tierische und pflanzliche Wachse sind Wachse im engeren Sinne, sie zählen zu den Lipiden. Hauptkomponenten dieser Stoffgemische sind in der Regel Ester von Fettsäuren (auch Wachssäuren genannt) mit langkettigen, aliphatischen, primären Alkoholen, den so genannten Fettalkoholen. Diese Ester unterscheiden sich in ihrem Aufbau von den Fetten und fetten Ölen, die Triglyceride mit Fettsäuren sind. Außerdem enthalten diese Wachse noch freie, langkettige, aliphatische Carbonsäuren, Ketone, Alkohole und Kohlenwasserstoffe (Wikipedia).

Das erfindungsgemäß eingesetzte Wachs ist Bienenwachs.

Bevorzugt ist das fette Ol Erdnussöl, Mandelöl, Sonnenblumenöl, Leinöl, Olivenöl oder Nachtkerzenöl. Mandelöl ist bevorzugt.

Die erfindungsgemäße Zusammensetzung umfasst einen zusätzlichen amphiphilen Nanocarrier als Emulgator, wie in den Ansprüchen definiert, Es wurde überraschend gefunden, dass der Wirkstoff selbst bereits als Emulgator wirkt und die Zugabe eines zusätzlichen Emulgators daher nicht nötig ist. Es kann jedoch auch ein weiterer Emulgator hinzugefügt werden. Ein solcher zusätzlicher Emulgator kann Polyvinylpyrrolidon Cetylstearylalkohol, Propylenglykol oder eine Kombination davon sein. Auch PEG kann als zusätzlicher Emulgator wirken. Cetylstearylalkohol ist ein Gemisch der Alkohole Cetylalkohol (Hexadecanol) und Stearylalkohol (Octadecanol), welches die Stabilität von Emulsionen erhöht und die Textur von Zubereitungen verbessern kann.

Der Emulgator wirkt bevorzugt auch als Penetrationsverbesserer oder Schlepper. In einer bevorzugten Ausführungsform ist der Emulgator Polyvinylpyrrolidon bevorzugt mit einem Molekulargewicht von etwa 2 500-100 000 Da, 5 000-40 000 Da, insbesondere etwa 8 000-20 000 Da oder 10 000 bis 15 000 Da. Bevorzugt wird Propylenglycol vermieden oder im Wesentlichen vermieden, da dieses v.a. in größeren Konzentrationen zu Hautreizungen führen kann.

Erfindungsgemäß wird als Emulgator bzw. Penetrationsmodulator ein amphiphiler Nanocarrier eingesetzt, insbesondere ein unimolekularer Nanocarrier mit dendritischer Struktur, wobei der Nanocarrier bevorzugt aus einem dendritischen Kern und aus mindestens zwei Schalen besteht, wobei eine innere Schale mit einem ersten Linker mit dem dendritischen Kern verbunden ist und eine äußere Schale mit einem zweiten Linker mit der inneren Schale verbunden ist, wie in den Ansprüchen definiert. In einer Ausführungsform sind die Nanocarrier neutral oder anionisch. Entsprechende Nanocarrier sind z.B. in WO 2006/018295 A2 offenbart. Auch Nanocarrier, die in Radowski et al. (Angew. Chem. Int. Ed. 2007, 46, 1265-1269); Fleige et al. (Macromolecules 2012, 45, 9452-9459), Fleige et al. (Nanocarriers, Vol. 1, 2013, 1-9, WO2011/095311), oder Haag et al., (Macromolecules, 2000, 33, 8158-8166) offenbart sind, können im Rahmen der Erfindung verwendet werden. Ein unimolekularer sulfatierter polyanionischer Nanocarrier, insbesondere eine unimolekulare polyanionische Polyglycerol-Micelle mit einer hydophilen Schale und einem hydrophoben Kern (EP-Anmeldung Nr. 14 161 579.9) kann ebenfalls eingesetzt werden.

Der Nanocarrier kann so ausgestaltet sein, dass
a) der dendritische Kern des Nanocarriers aus Polyglycerol ist, bevorzugt mit einem Molekulargewicht von 3-20 kDa, mehr bevorzugt 7-10 oder 8-9 kDa; und/oder
b) die innere Schale des Nanocarriers eine bevorzugt lineare Alkylkette mit einer Kohlenstofflänge von C2 bis C40, bevorzugt C8-C14 oder C10-C12 ist; und/oder
c) die äußere Schale Polyethylenglykol mit der Strukturformel (-CH₂-CH₂O-)ₙ mit n=3-130, welches endständig eine Methylgruppe, eine Hydroxylgruppe oder eine Carboxylgruppe, bevorzugt eine Methylgruppe, trägt, ist; und/oder
d) der erste Linker eine Ester oder Amidbindung ist; und/oder
e) der zweite Linker eine Esterbindung ist,
wobei bevorzugt alle Merkmale a-e zutreffen.

Der Nanocarrier kann dendritisches Polyglyceroldodecansäurepolyethylenglycolat umfassen. Er kann beispielhaft wie folgt charakterisiert sein:
Nomenklaturformel: hPG₁₀₀₀₀(-NH₂)_{0.7}(C₁₂mPEG₃₅₀)_{1.0 (Mn = 350)}
Alternative Nomenklatur: hPG₁₀₀₀₀(-NH₂)_{0.7}(C₁₂mPEG₆)_{1.0 (6 Wiederholungseinheiten im Mittel)}

hPG10k hat ca. 135 funktionelle Gruppen, davon können ca. 40-80%, z.B. etwa 70% zu Aminen umgesetzt sein (Indexzahl 0.7).

| **Molekülbestandteile von innen nach außen:** | **chemischer Bestandteil Größe** | **Synthese-Baustein** |
|---|---|---|
| 1. Dendritischer Kern | hyperverzweigtes Polyglycerin = hPG | hyperverzweigtes Polyglycerolamin = hPG(-NH₂) |
| | Mₙ = 8000-10000 Da | (Mₙ = 10 kDa, 40-80% Aminierung, insbesondere 65-70 %) |
| | *Peptidbindung* | |
| 2. Lipophile unpolare Schicht aus gesättigten Fettsäuren | C₁₀ oder C₁₂-Fettsäurekette, insbesondere C₁₂ | C₁₀-Disäure: |
| | | 1,10-decansäure bzw. |
| | | C₁₂-Disäure: |
| | | 1,12-dodecansäure |
| | *Esterbindung* | |
| 3. Hydrophile Polyethylenglykol kette | mPEG = Methoxypolyethylenglycol; | mPEG = Methoxypolyethylenglycol; |
| | Mₙ = 350 | Mₙ = 350, 500, 700 oder 1000, insbesondere 350 |

Der Nanocarrier kann aggregieren und bei dieser Aggregation Gastmoleküle (z.B. den Wirkstoff) aufnehmen. Unimere und Aggregate stehen im Gleichgewicht, welches sich mit zunehmender Verdünnung Richtung Unimer verschiebt, allerdings mit einer langsamen Kinetik. Zusätzlich kommt es, vor allem abhängig von Polarität und pH-Wert der Umgebung, zu einer Freisetzung der Gastmoleküle aus den Aggregaten. Dadurch kann der Nanocarrier nicht nur als Emulgator, sondern auch als Permeationsmodulator wirken, indem er zum einen die Kinetik der Wirkstofffreisetzung verbessern kann und zum anderen dabei helfen kann, die dermale Barriere zu überwinden Durch eine Wechselwirkung mit dem Stratum Corneum kann weiterhin eine optimale Einlagerung in obere Hautschichten mit nachfolgendem günstigen release-profil erreicht werden. Bevorzugt weist eine erfindungsgemäße Zusammensetzung 2,5-15 % w/w Thiamazol auf, bevorzugt ca. 10-15 % w/w oder 11-12% w/w Thiamazol oder ca. 5 bis 20 % w/w Carbimazol. Die bevorzugte Konzentration an Carbimazol kann ca. 1,6-2 fach so hoch sein wie die bevorzugte Thiamazol-Menge, oder es wird die ca. 1.6-2 fache Menge verabreicht.

In einer bevorzugten Ausführungsform umfasst eine erfindungsgemäße Zusammensetzung
a) 2,5-15 % w/w Thiamazol und/oder 5-20 % w/w Carbimazol, bevorzugt Thiamazol;
b) 5-18% w/w Wachs, bevorzugt ca. 12-16% w/w oder ca. 14,3% w/w;
c) 45-70% w/w fettes Öl, bevorzugt ca. 60-70% oder ca. 67,5% w/w;
d) 0-25% w/w Emulgator,.

Da der Emulgator ein Nanocarrier ist, kann die erfindungsgemäße Zusammensetzung z.B. 0,05-25% (w/w) des Nanocarriers umfassen, bevorzugt ca. 1-5% (w/w), ca. 0,25-3,5% (w/w) oder ca. 0,5 % (w/w). Bei anderen Emulgatoren, z.B. PVP, ist die Konzentration bevorzugt höher, z.B. 1-10%, bevorzugt ca.5-8% w/w oder ca. 6.5-7% w/w. Selbstverständlich können auch die Nanocarrier und andere Emulgatoren, z.B. PVP, kombiniert werden, z.B. in den genannten Mengen.

Die Zusammenfassung kann weitere Hilfsstoffe, Wirkstoffe, z.B. Dexpanthenol, oder Träger umfassen, z.B. Wasser oder Ethanol, dies ist jedoch nicht nötig. Die Zusammensetzung kann also wasserfrei sein. Bevorzugt ist die Zusammensetzung frei von Wasser, PEG und Propylenglykol.

Eine Zugabe von einem Tocopherol Derivat (Vitamin E oder antioxidativ wirksame Derivate hiervon), z.B. in einer Konzentration von 0.01-0,5%, bevorzugt 0.02-0,2% w/w, oder einem anderen hautfreundlichen Antioxidans ist bevorzugt.

In einer Ausführungsform ist die erfindungsgemäße Zusammensetzung ein Fettstift oder umfasst diesen. Bei Anwendung eines Fettstifts zur Verabreichung wird der Kontakt zur Haut des Anwenders (z.B. des Katzenhalters oder Pflegepersonals) minimiert, so dass bevorzugt Schutzmaßnahmen wie Handschuhe oder Fingerlinge nicht mehr benötigt werden. Dies erleichtert die Anwendung und erhöht, gerade bei Behandlung von Tieren wie Katzen, die Compliance. Es wurde gefunden, dass Tiere wie Katzen das Auftragen eines Fettstifts sehr gut tolerieren.

Ein erfindungsgemäßer Fettstift ist bevorzugt fest oder hochviskos und lässt sich mit leichtem Druck auf Haut auftragen. Der Fettstift ist bevorzugt thixotrop.

Die erfindungsgemäße Zusammensetzung, z.B. der Fettstift, kann in einer Halte- und Dosiervorrichtung (Applikator) enthalten oder fixiert sein, die ähnlich wie eine Lippenstifthülse aufgebaut sein kann. Mit der Vorrichtung kann die Zusammensetzung z.B. durch eine bestimmte Drehung um eine definierte Länge vorgeschoben werden, die einer transdermal aufzutragenden Dosis entspricht. Bevorzugt ist das Vollenden dieser bestimmten Drehung für den Drehenden spürbar oder sichtbar markiert, z.B. durch einen Widerstand beim Drehen oder eine optische Markierung.

Die erfindungsgemäße Zusammensetzung kann ein Gel oder eine Salbe oder Creme sein. Es kann sich um eine Wasser-in-ÖI-Suspension oder Öl-in-Wasser-Suspension handeln, in einer bevorzugten Ausführungsform ist die Zusammensetzung jedoch wasserfrei, mehr bevorzugt lipophil und wasserfrei. Gel, Salbe oder Creme sind halbfest und streichfähig, bevorzugt auch thixotrop. In einer bevorzugten Ausführungsform sind Gel, Salbe oder Creme in einem Applikator enthalten, wobei die Zusammensetzung z.B. durch Drehung, in geeigneter Dosis aus dem Applikator geschoben werden kann (z.B. 35-40 µl/Dosis). Es kann sich um einen Multidosisapplikator füllen. Der Applikator kann einen Aufsatz zur Verabreichung in der Innenseite der Ohrmuschel umfassen, der z.B. Pinselform aufweist. Die Zusammensatzung kann auch mit einem Handschuh/Fingerling aufgetragen werden.

Eine erfindungsgemäß formulierte Zusammensetzung, z.B. ein Fettstift oder bevorzugt eine lipophile wasserfreie Zusammensetzung, umfasst bevorzugt Thiamazol, Bienenwachs, und ein fettes Öl wie Mandelöl, optional ferner PVP und/oder einen Nanocarrier. Auch Mischungen verschiedener Wachse, insbesondere von flüssigen, halbfesten und/oder festen PEG können eingesetzt werden, um eine geeignete Viskosität zu erreichen. Beispielhafte Basisformulierungen, denen erfindungsgemäß noch Wirkstoff und optional Emulgator zugegeben werden, sind z.B. in WO/2008/009562 oder aus der Herstellung von Lippenstiften bzw. viskosen lipophilen Formulierungen offenbart. Ein Farbstoff/Pigment muss nicht enthalten sein.

Die erfindungsgemäße Zusammensetzung ist bevorzugt eine pharmazeutische Zusammensetzung, insbesondere eine zur transdermalen Verabreichung formulierte pharmazeutische Zusammensetzung.

Gegenstand der Erfindung ist auch die erfindungsgemäße Zusammensetzung zur Verwendung bei der Behandlung von Hyperthyreose. Gegenstand der Erfindung ist auch ein Verfahren zur Behandlung der Hyperthyreose, bei dem man eine erfindungsgemäße Zusammensetzung einem Patienten transdermal verabreicht.

Erfindungsgemäß kann die Hyperthyreose bei einer Katze behandelt werden. Alternativ ist der zu behandelnde Patient ein Mensch, ein Hund, ein Pferd, ein Kamel, ein Schwein, ein Rind, ein Kaninchen, ein Meerschweinchen, eine Maus oder eine Ratte.

Die Zusammensetzung kann zur topischen Verabreichung in der inneren Ohrmuschel formuliert sein, insbesondere in der inneren Ohrmuschel einer Katze.

Besondere Vorteile hat die erfindungsgemäße transdermale Zusammensetzung auch in der Behandlung der Hyperthyreose bei älteren und/oder multimorbiden Menschen, da auch hier Nebenwirkungen der oralen Gabe der Wirkstoffe vermieden werden können. Eine transdermale Gabe ist bei Menschen z.B. an der Innenseite des Unterarms unkompliziert möglich und erlaubt eine Selbstmedikation oder Verabreichung durch Dritte wie Pflegepersonal.

Dier erfindungsgemäße Zusammensetzung kann alle zwei Tage, täglich oder zweimal täglich eingesetzt werden. Eine Langzeittherapie (über mehrere Monate oder Jahre) oder eine medikamentöse Therapie vor einer Bestrahlungstherapie oder v.a. bei Menschen vor einer Thyroidektomie ist möglich.

Gegenstand der Erfindung ist auch eine Zusammensetzung zur transdermalen Verabreichung eines Wirkstoffs, nämlich Thiamazol oder Carbimazol, an eine Katze, wobei die Zusammensetzung ein Stift ist, insbesondere ein erfindungsgemäß beschriebener Fettstift. Gegenstand der Erfindung ist auch eine Zusammensetzung zur transdermalen Verabreichung eines Wirkstoffs, nämlich Thiamazol oder Carbimazol, an eine Katze, wobei die Zusammensetzung eine Creme ist, die in einem Applikator zur Verabreichung in der Ohrmuschel.enthalten ist. Bevorzugt ist der Applikator ein Multidosisapplikator.

Die Erfinder haben festgestellt, dass die erfindungsgemäße Zusammensetzung im Vergleich zu Zusammensetzungen aus dem Stand der Technik (US 2010/0137389 A1) eine signifikante Verringerung der therapeutischen Wirkstoffmenge ermöglicht. Dazu trägt z.B. die langanhaltende Freisetzung des Wirkstoffes bei.

Die Erfindung wird im Folgenden mit Beispielen illustriert, die zum Verständnis der Erfindung beitragen sollen, diese aber nicht beschränken sollen. "Ein" bedeutet im Rahmen der Erfindung immer "ein oder mehrere", soweit dies nicht explizit ausgeschlossen ist.

### Beispiele

### Beispiel 1 - Herstellung verschiedener Zusammensetzungen (Vergleichsbeispiele)

| | **Z1** | | **Z2** | | **Z3** | |
|---|---|---|---|---|---|---|
| **Wirkstoff** | Thiamazol | 11,2% | Thiamazol | 11,2% | Thiamazol | 11,2% |
| **Wachs** | Cera alba | 14,3% | Cera alba | 14,3% | Cera alba | 15,8% |
| **Fettes Öl** | Mandelöl | 67,5% | Mandelöl | 68,0% | Mandelöl | 72,5% |
| **Emulgator** | PVP | 6,5% | PVP | 6,5% | Nanocarrier | 0,5% |
| | Nanocarrier | 0,5% | | | | |

| | **Z4** | | **Z5** | | **Z6** | |
|---|---|---|---|---|---|---|
| **Wirkstoff** | Thiamazol | 11,2% | Thiamazol | 11,2% | Thiamazol | 11,2% |
| **Wachs** | Cera alba | 16,0% | Cetylpalmitat | 14,0% | Cera alba | 15,8% |
| **Fettes Öl** | Mandelöl | 72,8% | Mineralöl | 74,8% | Mineralöl | 72,5% |
| **Emulgator** | | 0% | | 0% | Nanocarrier | 0,5% |

| | | | | | | |
|---|---|---|---|---|---|---|
| %: w/w Nanocarrier: Polyglyceroldodecansäurepolyethylenglycolat. PVP: Molekulargewicht von 10.000 bis 17.000 Da. | | | | | | |

| | **Z7** | | **Z8** | | **Z9** | |
|---|---|---|---|---|---|---|
| **Wirkstoff** | Thiamazol | 11.2% | Thiamazol | 7,6% | Thiamazol | 11.2% |
| **Wachs** | Cera alba | 14,3% | Cera alba | 13.4% | Cera alba | 14.3 % |
| **Fettes Öl** | Mandelöl | 67.5% | Mandelöl | 72,0% | Mandelöl | 67,5% |
| **Emulgator** | PVP | 6,5% | PVP | 6,5% | PVP | 6,5% |
| | Nanocarrier | 0,5% | Nanocarrier | 0.5 % | Nanocarrier | 0.0 % |

### Beispiel 2:

Eine 14 Jahre alte, kastrierte, hyperthyreotrophe Katze wurde mit einer Formulierung vom Typ Z7 und Z8 sowie Z9 behandelt. Der TT4 Wert zu Beginn der Behandlung betrug 23.7. Nach 4 Wochen Behandlung mit einer Dosis von 100 µl vom Formulierungstyp Z7 sank der TT4 Wert auf einen Wert von 8.7 und das Befinden der Katze besserte sich signifikant. Nach einer Dosiserhöhung auf 14 mg / Tag durch 2maliges Auftragen vom Typ Z8 in den unteren euthyreotischen Bereich ab. Anschließend wurde eine Dosisanpassung auf 11.2 mg pro Tag (Typ Z9) vorgenommen, um einen stabilen Wert im mittleren bis oberen euthyreoten Bereich zu erreichen. Die gesamte Behandlungsdauer betrug 270 Tage.

### Beispiel 3:

Zwei hyperthyreotische Katzen wurden mit einer lipophilen, wasserfreien Formulierung vom Typ Z7 behandelt. Die Dosis betrug 5 mg/Tag. Beide Katzen besaßen vor der Behandlung einen TT4 Wert von 8.6 +-0.5, nach 14 Tagen Behandlung einen TT4 Wert von 3.7-4.3.

### Beispiel 4:

Vier hyperthyreoten Katzen wurde eine lipophile, wasserfreie Formulierung vom Typ Beispiel Z9 appliziert. Die Anfangsdosis betrug 5,5 mg / Tag und wurde nach 28 Tagen auf 3,5 mg Tag bis 5.5 mg individuell angepasst. Der mittlere TT4 Wert der Katzen betrug vor der Behandlung 9.81.

Nach 7 bis 14 Tagen betrug der mittlere TT4 Wert 5.7, nach 90 Tagen 4.5. Die Katzen befanden sich damit im eurthyreoten Bereich, der zwischen 4.5 und 1.0 liegt.

### Beispiel 5:

Vier hyperthyreoten Katzen wurde eine Formulierung vom Typ Z9 über 10 bis 14 Tage appliziert. Bei allen Katzen wurde eine signifikante Absenkung des TT4 Wertes beobachtet (von ursprünglich 9,8 +- 2,2 auf 7,4 +- 2,5). Hierbei betrug die Dosis 5,5 mg alle 2 Tage. Die TT4 Werte an behandlungsfreien Tagen unterschieden sich nicht signifikant von den TT4 Werten an Tagen, an denen behandelt wurde. Dies legt überraschenderweise nahe, dass die in der Erfindung beschriebenen wasserfreien Formulierungstypen bei ausreichender Dosisstärke auch als Retardformulierung einsetzbar sind.

### Beispiel 6:

Die Formulierungen vom Typ Z7 wurden über 90 Tage bei 30°C, 40°C sowie 50°C gelagert. Es wurde keine Absenkung des Thiamazolgehaltes beobachtet.

### Beispiel 7:

22.4g Thiamazol und 13.0 g PVP wurden in einer Fantaschale mit einer kleinen Menge (ca. 5g) Oleum amygdalarum (Mandelöl) angerieben. 28.60 g Cera Alba, 1,0 g Nanocarrier sowie 130 g wurden im Wasserbad aufgeschmolzen. Anschließend wurde unter Rühren auf Raumtemperatur abgekühlt und dabei die Thiamazol/PVP Mischung in den Ansatz eingearbeitet.

### Beispiel 8:

Wirkstoff Thiamazol wurde mit der 30 g Mandelöl sowie optional dem Nanocarrier angerieben. 30 g Mandelöl wurden zusammen mit Bienenwachs und Mandelöl im Wasserbad geschmolzen und anschließend kaltgerührt. Anschließend wurde der Wirkstoff in die Salbengrundlage eingearbeitet.

## Patentansprüche

1. Zusammensetzung, umfassend
a) einen Wirkstoff ausgewählt aus der Gruppe bestehend aus Thiamazol und/oder Carbimazol, wobei die Zusammensetzung 2,5-15 % w/w Thiamazol und/oder 5-20 % w/w Carbimazol umfasst;
b) 5-18% w/w Bienenwachs; und
c) 45-75% w/w von mindestens einem fetten Öl, ausgewählt aus der Gruppe bestehend aus Mandelöl, Erdnussöl, Sonnenblumenöl, Leinöl, Olivenöl, oder Nachtkerzenöl,
d) 0,25-3,5% w/w eines amphiphilen unimolekularen Nanocarriers mit dendritischer Struktur, welcher aus einem dendritischen Kern und aus mindestens zwei Schalen besteht, wobei eine innere Schale mit einem ersten Linker mit dem dendritischen Kern verbunden ist, und eine äußere Schale mit einem zweiten Linker mit der inneren Schale verbunden ist, wobei
i) der dendritische Kern des Nanocarriers aus Polyglycerol ist; und/oder
ii) die innere Schale des Nanocarriers eine Alkylkette mit einer Kohlenstofflänge von C2 bis C40, ist; und/oder
iii) die äußere Schale Polyethylenglykol mit der Strukturformel (-CH₂-CH₂O-)ₙ mit n=3-130, welches endständig eine Methylgruppe, eine Hydroxylgruppe oder eine Carboxylgruppe, trägt, ist; und/oder
iv) der erste Linker eine Ester oder Amidbindung ist; und/oder
v) der zweite Linker eine Esterbindung ist,
wobei bevorzugt alle Merkmale i-v zutreffen.

2. Zusammensetzung nach Anspruch 1, wobei die Zusammensetzung wasserfrei ist.

3. Zusammensetzung nach einem der vorhergehenden Ansprüche, wobei der Wirkstoff Thiamazol oder Carbimazol ist.

4. Zusammensetzung nach einem der vorhergehenden Ansprüche, wobei das fette Öl Mandelöl ist.

5. Zusammensetzung nach einem der vorhergehenden Ansprüche, welche ferner mindestens einen Emulgator umfasst, der ausgewählt ist aus der Gruppe umfassend Polyvinylpyrrolidon Polyethylenglykol, Cetylstearylalkohol, und Propylenglykol.

6. Zusammensetzung nach einem der Ansprüche 1-5, wobei
a) der dendritische Kern des Nanocarriers mit einem Molekulargewicht von 3-20 kDa aus Polyglycerol ist, bevorzugt 7-10 oder 8-9 kDa; und
b) die innere Schale des Nanocarriers eine lineare Alkylkette mit einer Kohlenstofflänge von C2 bis C40, bevorzugt C8-C14 oder C10-C12 ist; und
c) die äußere Schale Polyethylenglykol mit der Strukturformel (-CH₂-CH₂O-)ₙ mit n=3-130, welches endständig eine Methylgruppe, eine Hydroxylgruppe oder eine Carboxylgruppe, bevorzugt eine Methylgruppe, trägt, ist; und
d) der erste Linker eine Ester oder Amidbindung ist; und
e) der zweite Linker eine Esterbindung ist.

7. Zusammensetzung nach einem Ansprüche 5 bis 6, wobei der Emulgator Polyvinylpyrrolidon ist, bevorzugt mit einem Molekulargewicht von etwa 5 000-40 000 Da, insbesondere etwa 7000-17000 Da, etwa 8000-15000 Da oder etwa 10000 Da.

8. Zusammensetzung nach einem der vorhergehenden Ansprüche, welche 2,5-15 % w/w Thiamazol und/oder Carbimazol umfasst, bevorzugt ca. 10-12 % w/w Thiamazol oder ca. 11 bis 11,2 % w/w Thiamazol.

9. Zusammensetzung nach einem der vorhergehenden Ansprüche, umfassend
b) 11-15% w/w Wachs, bevorzugt 14-15% w/w oder ca. 14,3% w/w;
c) 60-70% w/w fettes Öl, bevorzugt ca. 65-75% w/w oder ca. 67,5% w/w;
d) 0,25-25% w/w Emulgator, bevorzugt ca. 0,25-10%, ca. 5-8% w/w oder ca. 6.5-7% w/w.

10. Zusammensetzung nach einem der vorhergehenden Ansprüche, welche Salbe in einem geeigneten Applikator ist oder diesen umfasst, wobei die Zusammensetzung bevorzugt Thiamazol, Bienenwachs und ein fettes Öl wie Mandelöl umfasst.

11. Zusammensetzung nach einem der vorhergehenden Ansprüche, welche eine pharmazeutische Zusammensetzung, bevorzugt eine zur transdermalen Verabreichung formulierte pharmazeutische Zusammensetzung ist.

12. Zusammensetzung nach einem der vorhergehenden Ansprüche zur Verwendung bei der Behandlung von Hyperthyreose, wobei bevorzugt die Hyperthyreose bei einer Katze behandelt wird.

13. Zusammensetzung nach einem der Ansprüche 11 oder 12, wobei die Zusammensetzung zur topischen Verabreichung in der inneren Ohrmuschel ist, insbesondere in der inneren Ohrmuschel einer Katze.

14. Multidosisapplikator, umfassend eine Zusammensetzung nach einem der vorhergehenden Ansprüche zur Verwendung bei der Behandlung der Hyperthyreose einer Katze, wobei die Zusammensetzung ein Gel, eine Salbe oder Creme ist und transdermal verabreicht wird, wobei der Applikator einen Aufsatz zur Verabreichung in der Innenseite der Ohrmuschel umfasst, der Pinselform aufweist.

## Claims

1. A composition comprising
a) an active agent selected from the group consisting of thiamazole and/or carbimazole, wherein the composition comprises 2.5-15% (w/w) of thiamazole and/or 5-20% (w/w) of carbimazole;
b) 5-18% (w/w) of beeswax; and
c) 45-75% (w/w) of at least one fatty oil selected from the group consisting of almond oil, peanut oil, sunflower oil, linseed oil, olive oil, or evening primrose oil,
d) 0.25-3.5% (w/w) of an amphiphilic unimolecular nanocarrier of dendritic structure, composed of a dendritic core and at least two shells, wherein an inner shell is coupled to the dendritic core via a first linker, and an outer shell is coupled to the inner shell via a second linker, wherein
i) the dendritic core of the nanocarrier is made of polyglycerol, and/or
ii) the inner shell of the nanocarrier is an alkyl chain with a carbon length of C2 to C40; and/or
iii) the outer shell is polyethylene glycol having the structural formula (CH₂CH₂O)ₙ with n = 3-130, which bears a terminal methyl group, a hydroxyl group or a carboxyl group; and/or
iv) the first linker is an ester or amide bond, and/or
v) the second linker is an ester bond,
wherein preferably all features i-v apply.

2. The composition according to claim 1, wherein the composition is free of water.

3. The composition according to any of the previous claims, wherein the active agent is thiamazole or carbimazole.

4. The composition according to any of the previous claims, wherein the fatty oil is almond oil.

5. The composition according to any of the previous claims, further comprising at least one emulsifier selected from the group comprising polyvinylpyrrolidone, polyethylene glycol, cetyl stearyl alcohol and propylene glycol.

6. The composition according to any of claims 1-5, wherein
a) the dendritic core of the nanocarrier with a molecular weight of 3-20 kDa is made of polyglycerol, preferably 7-10 or 8-9 kDa; and
b) the inner shell of the nanocarrier is a linear alkyl chain with a carbon length of C2 to C40, preferably C8-C14 or C10-C12; and
c) the outer shell is polyethylene glycol having the structural formula (CH₂CH₂O)ₙ with n = 3-130, which bears a terminal methyl group, a hydroxyl group or a carboxyl group, preferably a methyl group; and
d) the first linker is an ester or amide bond; and
e) the second linker is an ester bond.

7. The composition according to any of claims 5-6, wherein the emulsifier is polyvinylpyrrolidone, preferably with a molecular weight of about 5 000-40 000 Da, in particular about 7 000-17 000 Da, about 8 000-15 000 or about 10 000 Da.

8. The composition according to any of the previous claims, comprising 2.5-15% (w/w) of thiamazole and/or carbimazole, preferably about 10-12% (w/w) of thiamazole or about 11 to 11.2% (w/w) of thiamazole.

9. The composition according to any of the previous claims, comprising
b) 11-15% (w/w) of wax, preferably 14-15% (w/w) or about 14.3% (w/w);
c) 60-70% (w/w) of fatty oil, preferably about 65-75% (w/w) or about 67.5% (w/w);
d) 0.25-25% (w/w) of emulsifier, preferably about 0.25.-10%, about 5-8% (w/w) or about 6.5-7% (w/w).

10. The composition according to any of the previous claims, which is an ointment in a suitable applicator or comprises the same, wherein the composition preferably comprises thiamazole, beeswax and a fatty oil such as almond oil.

11. The composition according to any of the previous claims, which is a pharmaceutical composition, preferably a pharmaceutical composition formulated for transdermal administration.

12. The composition according to any of the previous claims for use in treatment of hyperthyreosis, wherein preferably the hyperthyreosis of a cat is treated.

13. The composition according to any of the claims 11 or 12, wherein the composition is for the topical administration on the inside of the pinna, especially on the inside of the pinna of a cat.

14. An applicator with multiple dosage units, comprising the composition according to any of the previous claims for use in treatment of hyperthyreosis of a cat, wherein the composition is a gel, an ointment or a cream and is administered transdermally, wherein the applicator comprises an attachment for administration on the inside of the pinna, having a brush shape.

## Revendications

1. Composition, comprenant
a) une substance active choisie dans l'ensemble constitué par le thiamazole et/ou le carbimazole, la composition comprenant 2,5 à 15 % p/p de thiamazole et/ou 5 à 20 % p/p de carbimazole ;
b) 5-18 % p/p de cire d'abeille ; et
c) 45-75 % p/p d'au moins une huile grasse, choisie dans l'ensemble constitué par l'huile d'amande, l'huile d'arachide, l'huile de tournesol l'huile de lin, l'huile d'olive, ou l'huile d'onagre,
d) 0,25-3,5 % p/p d'un nanovéhicule unimoléculaire amphiphile à structure dendritique, qui est constitué d'un noyau dendritique et d'au moins deux enveloppes, une enveloppe interne étant liée au noyau dendritique par un premier segment de liaison, et une enveloppe externe étant liée à l'enveloppe interne par un second segment de liaison, où
i) le noyau dendritique du nanovéhicule est constitué de polyglycérol ; et/ou
ii) l'enveloppe interne du nanovéhicule est une chaîne alkyle ayant une longueur en atomes de carbone de C2 à C40 ; et/ou
iii) l'enveloppe externe est un polyéthylèneglycol ayant la formule structurale (-CH₂-CH₂O-)ₙ où n = 3-130, qui porte en bout de chaîne un groupe méthyle, un groupe hydroxy ou un groupe carboxy ; et/ou
iv) le premier segment de liaison est une liaison ester ou amide ; et/ou
v) le second segment de liaison est une liaison ester,
de préférence toutes les caractéristiques i-v étant valables.

2. Composition selon la revendication 1, dans laquelle la composition est anhydre.

3. Composition selon l'une quelconque des revendications précédentes, dans laquelle la substance active est le thiamazole ou le carbimazole.

4. Composition selon l'une quelconque des revendications précédentes, dans laquelle l'huile grasse est l'huile d'amande.

5. Composition selon l'une quelconque des revendications précédentes, qui comprend en outre au moins un émulsifiant, qui est choisi dans l'ensemble comprenant la polyvinylpyrrolidone, le polyéthylèneglycol, l'alcool cétylstéarylique, et le propylèneglycol.

6. Composition selon l'une quelconque des revendications 1 à 5, dans laquelle
a) le noyau dendritique du nanovéhicule est constitué de polyglycérol ayant une masse moléculaire de 3-20 kDa, de préférence 7-10 ou 8-9 kDa ; et
b) l'enveloppe interne du nanovéhicule est une chaîne alkyle linéaire ayant une longueur en atomes de carbone de C2 à C40, de préférence C8 à C14 ou C10 à C12 ; et
c) l'enveloppe externe est un polyéthylèneglycol ayant la formule structurale (-CH₂-CH₂O-)ₙ où n = 3-130, qui porte en bout de chaîne un groupe méthyle, un groupe hydroxy ou un groupe carboxy, de préférence un groupe méthyle ; et
iv) le premier segment de liaison est une liaison ester ou amide ; et
v) le second segment de liaison est une liaison ester.

7. Composition selon l'une quelconque des revendications 5 et 6, dans laquelle l'émulsifiant est la polyvinylpyrrolidone, de préférence ayant une masse moléculaire d'environ 5 000 à 40 000, en particulier d'environ 7 000 à 17 000 Da, environ 8 000 à 15 000 Da ou environ 10 000 Da.

8. Composition selon l'une quelconque des revendications précédentes, qui comprend 2,5 à 15 % p/p de thiamazole et/ou carbimazole, de préférence environ 10 à 12 % p/p de thiamazole ou environ 11 à 11,2 % p/p de thiamazole.

9. Composition selon l'une quelconque des revendications précédentes, comprenant
b) 11-15 % p/p, de préférence 14-15 % p/p ou environ 14,3 % p/p de cire ;
c) 60-70 % p/p, de préférence 65-75 % p/p, ou environ 67,5 % p/p d'huile grasse ;
d) 0,25-25 % p/p, de préférence environ 0,25-10 % p/p, environ 5-8 % p/p ou environ 6,5-7 % p/p d'émulsifiant.

10. Composition selon l'une quelconque des revendications précédentes, qui est une pommade dans un applicateur approprié ou comprend ce dernier, la composition comprenant de préférence du thiamazole, de la cire d'abeille et une huile grasse telle que l'huile d'amande.

11. Composition selon l'une quelconque des revendications précédentes, qui est une composition pharmaceutique, de préférence une composition pharmaceutique formulée pour l'administration transdermique.

12. Composition selon l'une quelconque des revendications précédentes, destinée à être utilisée dans le traitement de l'hyperthyroïdie, de préférence l'hyperthyroïdie étant traitée chez un chat.

13. Composition selon l'une quelconque des revendications 11 et 12, la composition étant destinée à l'administration topique sur la face interne du pavillon auriculaire, en particulier sur la face interne du pavillon auriculaire d'un chat.

14. Applicateur multidose, comprenant une composition selon l'une quelconque des revendications précédentes, destiné à être utilisé dans le traitement de l'hyperthyroïdie d'un chat, la composition étant un gel, une pommade ou une crème et étant administrée par voie transdermique, l'applicateur comportant un embout destiné à l'administration dans la face interne du pavillon auriculaire, qui a la forme d'un pinceau.
